Europäisches Patentamt

European Patent Office

Office européen des brevets

(11) Veröffentlichungsnummer : **0 290 921 B1**

## (12) EUROPÄISCHE PATENTSCHRIFT

(45) Veröffentlichungstag der Patentschrift :
31.08.94 Patentblatt 94/35

(51) Int. Cl.⁵ : **G01N 33/52**

(21) Anmeldenummer : **88107065.0**

(22) Anmeldetag : **03.05.88**

(54) Testträger für die Analyse einer Probenflüssigkeit und Verfahren zu seiner Herstellung.

(30) Priorität : **09.05.87 DE 3715485**

(43) Veröffentlichungstag der Anmeldung :
**17.11.88 Patentblatt 88/46**

(45) Bekanntmachung des Hinweises auf die
Patenterteilung :
**31.08.94 Patentblatt 94/35**

(84) Benannte Vertragsstaaten :
**AT BE CH DE ES FR GB GR IT LI LU NL SE**

(56) Entgegenhaltungen :
EP-A- 0 016 387
EP-A- 0 192 320
EP-A- 0 299 428
DE-A- 1 598 859
GB-A- 1 420 916
US-A- 4 397 959
US-A- 4 407 943
JOURNAL OF IMMUNOLOGICAL METHODS,
vol. 85, no. 2, 1985, New York, NY (US); J.G.
KENNA et al., Seiten 409-419
JOURNAL OF IMMUNOLOGICAL METHODS,
vol. 88, no. 2, 1986, New York, NY (US); J.H.
SKERRITT et al., Seiten 217-224

(73) Patentinhaber : **BOEHRINGER MANNHEIM
GMBH
D-68298 Mannheim (DE)**

(72) Erfinder : **Freitag, Helmut, Dr.
9115 Hague Road
Indianapolis, IN 46250 (US)**
Erfinder : **Wilk, Hans-Erich, Dipl.Chem. Dr.
Goldregenstrasse 5
D-6143 Lorsch (DE)**
Erfinder : **Rothe, Anselm, Dr.
Tiefenklinger Weg 21
D-6943 Birkenau (DE)**
Erfinder : **Eikmeier, Heino, Dipl.Biol. Dr.
Bismarckstrasse 8
D-6143 Lorsch (DE)**

(74) Vertreter : **Pfeifer, Hans-Peter, Dr.rer.nat.
Patentanwalt
Nowackanlage 15
D-76137 Karlsruhe (DE)**

## Beschreibung

Die Erfindung betrifft einen Testträger für die Analyse einer Probenflüssigkeit mit einer porösen Testschicht, sowie ein Verfahren zur Herstellung eines solchen Testträgers.

Zur qualitativen oder quantitativen analytischen Bestimmung von Probenflüssigkeiten, insbesondere von Körperflüssigkeiten wie Blut oder Urin werden in jüngerer Zeit zunehmend sogenannte trägergebundene Tests verwendet. Bei diesen sind Reagenzien in mindestens einer Testschicht eingebettet, die mit der Probe in Kontakt gebracht wird. Die Reaktion von Probe und Reagenzien führt zu einem nachweisbaren Signal, insbesondere einem Farbumschlag.Dieser kann in einfachen Fällen visuell ausgewertet werden. Bei quantitativen Bestimmungen erfolgt die Auswertung überwiegend mit Hilfe eines Gerätes, insbesondere reflexionsphotometrisch.

Testträger sind häufig als Teststreifen ausgebildet, die im wesentlichen aus einer länglichen Tragschicht aus Kunststoffmaterial bestehen, auf der eine oder mehrere Testschichten angebracht sind. Es sind jedoch auch andere Testträgergestaltungen bekannt, beispielsweise in Form von quadratischen oder rechteckigen Plättchen.

Testträger und die zugehörigen Auswertegeräte, die insgesamt auch als Testträger-Analysesysteme bezeichnet werden, zeichnen sich gegenüber den vorbekannten naßchemischen Analyseverfahren vor allem durch einfache Handhabung und geringen apparativen Aufwand aus. Deswegen werden in zunehmendem Maße immer aufwendigere Analysen mit Hilfe von Testträgern durchgeführt. Dies führte zur Entwicklung von Testträgern, die mehrere Testschichten aufweisen. Die Testschichten sind auf einer Tragschicht übereinander oder nebeneinander so angeordnet, daß sich ein gewünschter Testablauf ergibt. Durch entsprechende konstruktive Maßnahmen kann sogar ein mehrstufiger Reaktionsablauf erreicht werden (DE-A 36 38 654).

Die Testschichten von Testträgern dienen vor allem als Reagenzträger. Trockene Reagenzien sind in eluierbarer oder in trägerfixierter Form in der Schicht enthalten, wobei eluierbare Reagenzien von der Probenflüssigkeit gelöst oder dispergiert werden und meist erst nach der Elution in der flüssigen Phase reagieren, während trägerfixierte Reagenzien in fixierter Form an der Reaktion teilnehmen.

Die Erfindung richtet sich speziell auf poröse Testträgerschichten. Für die Reagenzträgerfunktion ist die Porosität wegen der damit verbundenen großen Oberfläche meist vorteilhaft. Zugleich erfüllen poröse Schichten im allgemeinen eine Flüssigkeitstransportfunktion, die auf den in der Schicht wirkenden Kapillarkräften basiert.

Poröse Testschichten haben mindestens eine feste Komponente, die alleine oder mit anderen Komponenten eine dreidimensionale offenporige Struktur bildet. Die Struktur kann sehr verschieden sein.Bekannt sind beispielsweise Testschichtpapiere oder poröse Kunststoffstrukturen, die man auch als offenporige Membranen oder poröse Folien bezeichnen kann.

Für die Erfindung von besonderer Bedeutung sind textile Strukturen, beispielsweise Gewebe oder Vliese, bei denen die feste Komponente von miteinander verschlungenen Fäden oder Fasern gebildet wird. Selbstverständlich können dabei mehrere verschiedene Materialien eingesetzt werden, die verschiedene feste Komponenten bilden.

An Testschichten für moderne, quantitative Testträger werden sehr hohe Anforderungen gestellt. Dies gilt vor allem für immunchemische Verfahren.

Die bei derartigen Verfahren erforderlichen biochemischen Reagenzien, insbesondere Enzyme oder Enzymkonjugate müssen für die Reaktion im Zuge des Testablaufes in sehr exakt vorbestimmter Menge zur Verfügung stehen. Dies steht im Gegensatz zu den meisten klassischen klinisch-chemischen Verfahren, bei denen derartige Reagenzien meist im Überschuß verwendet werden, ohne daß dies einen wesentlichen Einfluß auf die Genauigkeit des Ergebnisses hat. Immunchemische Verfahren lassen sich deswegen im Regelfall auf festen Trägern nur durchführen, wenn die erforderlichen biochemischen Reagenzien

- in genau dosierter Menge auf einen Testträger aufgegeben werden können,
- ihre Enzymaktivität auch nach längerer Lagerung keinen Abfall aufweist und
- während der Lagerung bis zum Verbrauch durch den Anwender ein unverändertes Auflösungsverhalten zeigen.

Diese schwierigen Anforderungen werden von den bekannten Testschichten nicht in ausreichendem Maße erfüllt. Der Erfindung liegt daher die Aufgabe zugrunde, Testschichten für Testträger zur Verfügung zu stellen, die hinsichtlich der Reagenzträgerfunktion und/oder hinsichtlich der Transportfunktion verbesserte Eigenschaften bezüglich dieser Anforderungen haben.

Diese Aufgabe wird erfindungsgemäß durch einen Testträger gemäß Anspruch 1 gelöst.

Das Protein muß unter den Bedingungen, unter denen die Analyse auf dem Testträger abläuft, so weitgehend unlöslich sein, daß die Beschichtung der festen Komponente erhalten bleibt. Übliche analytische Bestimmungen auf Testträgern laufen bei pH-Werten zwischen etwa 5,5 und 8 und bei Temperaturen zwischen 20°C und 37°C ab. Als gut geeignet für die Erfindung hat sich Casein erwiesen, das unter diesen Bedingungen nahezu vollständig unlöslich ist. Ein anderes geeignetes Protein ist Edestin. Dessen Einsatzmöglichkeiten sind allerdings durch seine schwarze Farbe beschränkt. Die Eignung anderer unter den jeweiligen Testbedingungen unlöslicher Proteine kann der Fachmann ohne weiteres feststellen.

Um die feste Komponente mit dem Protein zu beschichten, wird dieses mit einer Lösung des Protein behandelt. Die Lösung wird unter Bedingungen hergestellt, unter denen die Löslichkeit des Proteins ausreichend hoch ist, um eine für die Beschichtung notwendige Mindestmenge zu lösen. Danach wird die feste Komponente mit der Lösung beaufschlagt und die Löslichkeit durch geeignete Maßnahmen so weit vermindert, daß zumindest ein Teil des Proteins ausfällt und die Komponente von dem ausfallenden Protein beschichtet wird.

Einzelheiten des Verfahrens hängen vom jeweiligen Einzelfall ab.

Beispielsweise kann die erforderliche Löslichkeit zur Herstellung der Behandlungslösung durch Wahl eines geeigneten Lösungsmittels erreicht werden, wobei der Lösung nach dem Kontaktieren mit der festen Komponente ein Fällungsmittel zugegeben wird, um die Beschichtung einzuleiten.

Man kann auch so vorgehen, daß man von einer stark verdünnten Lösung ausgeht und das Lösungsmittel verdampft, um die Präzipitation des Proteins einzuleiten. Selbstverständlich kann die Löslichkeit auch durch Veränderung der Temperatur im gewünschten Sinne beeinflußt werden.

Besonders bevorzugt verändert man die Löslichkeit durch eine entsprechende Änderung des pH-Wertes beispielsweise durch Zugabe von NaOH. Es hat sich beispielsweise gezeigt, daß Casein bei einem pH-Wert von mindestens 10,5 so weitgehend in Wasser löslich ist, daß eine ausreichende Proteinkonzentration erreicht werden kann. Das Ausfällen des Proteins wird in diesem Falle durch Umpufferung auf pH-Werte zwischen 5,5 und 8 erreicht. Dies führt zu einer hochwertigen Beschichtung der festen Komponente.

Die feste Komponente kann auf verschiedenerlei Weise mit der Proteinlösung kontaktiert werden. Vielfach ist es zweckmäßig, die gesamte Testschicht (also nicht nur eine oder mehrere feste Komponenten derselben) zu beschichten.

Dies gilt insbesondere für Testschichten mit textiler Struktur, wie z.B. Vliese oder Gewebe. Hier erfolgt die Kontaktierung durch Sprühen oder in dem bei Testträgerschichten weitgehend üblichen Tauchtränkverfahren.

Soweit nur einzelne Komponenten einer Testschicht mit dem unlöslichen Protein beschichtet werden sollen, muß dies geschehen, bevor sie in die Testschicht integriert werden.

Es ist nicht unbedingt erforderlich, daß die Proteinbeschichtung die Oberfläche der festen Komponente ganz vollständig bedeckt. Vielmehr ist je nach der Mikrostruktur des Materials, aus dem die feste Komponente besteht, damit zu rechnen, daß ein im allgemeinen geringer Teil der festen Oberfläche unbedeckt bleibt. Dies hat sich jedoch als unschädlich erwiesen.

Um eine ausreichende Bedeckung der Oberfläche zu erreichen, ist eine entsprechende Mindestmenge des Proteins, bezogen auf die Menge der zu beschichtenden festen Komponente, erforderlich. Diese läßt sich im Einzelfall experimentell bestimmen. Es hat sich gezeigt, daß ein Überschreiten dieser Menge im Regelfall unkritisch ist. Ein Überschuß führt überwiegend nicht zu einer Verstärkung der Beschichtung, sondern er fällt frei aus und kann deswegen ohne weiteres abgetrennt werden.

Die Konzentration des Proteins in der Beschichtungslösung kann in weiten Grenzen variieren. Praktisch bewährt haben sich Konzentrationen zwischen 0,1 und 1 %.

Die erfindungsgemäße Proteinbeschichtung erweist sich als besonders vorteilhaft, wenn die Testschicht ein biochemisches Reagenz in eluierbarer Form enthält. Das Reagenz kann insbesondere ein Enzym oder ein Enzymkonjugat (beispielsweise mit einem Antikörper oder Antigen) sein. Vor allen Dingen für Reagenzschichten, die das als Markierungsenzym in Immuntesten häufig verwendete Enzym Galactosidase enthalten, hat sich die Erfindung praktisch bewährt. Es wird erreicht, daß eine bestimmte bei der Herstellung des Testträgers in die Testschicht inkorporierte Reagenzmenge selbst nach jahrelanger Lagerung mit sehr guter Genauigkeit eluiert werden kann und damit für die Reaktion zur Verfügung steht. Vor allem in diesem Zusammenhang sind Testschichten mit einer textilen Struktur (insbesondere Gewebe oder Vliese) aus Polyester oder Polyamid besonders bevorzugt.

Unter β-Galactosidase ist das Enzym E.C. 3.2.1.23 zu verstehen. Unter β-Galactosidase-Konjugaten werden Verbindungen der Galactosidase mit Antikörpern (IgG sowie Fab), Haptenen, Proteinen usw. verstanden. Die Herstellung solcher Konjugate, die im Prinzip bekannt sind, geschieht nach jedem Fachmann geläufigen Techniken.

Im Zusammenhang mit der Imprägnierung von Testschichten mit Enzymen sind bereits Proteine, insbesondere Rinderserumalbumin, als sogenannte Schutzproteine eingesetzt worden. Sie wurden in großer Menge der Tränklösung zugesetzt, um die Enzyme in der Tränklösung zu stabilisieren.

In der EP-A-0 192 320 werden getrocknete Reagenzzubereitungen beschrieben, bei denen gelöstes Albumin mit einem Enzymkonjugat getrocknet wird, um die Lagerstabilität zu verbessern. Das Albumin wird vorzugsweise in einer reduzierten Konzentration von 1-3 % eingesetzt. Ein unter Testbedingungen unlösli- ches Protein wird nicht erwähnt.

In verschiedenen Publikationen (DE-B-1 598 859, GB-A-1 420 916 und US-A-4 407 943) wird die Möglichkeit erwähnt, im Zusammen- hang mit der Trägerfixierung immunologischer Reagenzbestandteile (Antikörper oder Antigen) an der Oberfläche ein Protein (insbesondere Albumin) aus einer Lösung zu adsorbieren, um eine verbesserte Bindung des Antigens bzw. des Antikörpers zu erreichen.

In dem Artikel von J.G. Kenna et al. "Methods for reducing non-specific antibody binding in enzyme-linked immunosorbent assays", J. of immunological methods 85 (1985), 409-419 wird Casein als Blockierungsmittel verwendet. Da es als Ersatz für lösliche Blockierungsmittel (Detergens Twen 20, Albumin) verwendet wird, muß es sich um ein lösliches Casein handeln. In der Tat wurde bei den Experimenten gemäß den in dem Artikel gemachten Angaben ein Casein "Hammarsten grade" verwendet, welches ausweislich entsprechender Kataloge (Serva-Katalog) in Wasser vollständig löslich ist.

Wichtig ist, daß die Proteinbeschichtung und das eluierbare Reagenz in zwei getrennten Schritten aufgebracht werden. Dies bedeutet zwar nicht, daß notwendigerweise zwei exakt voneinander getrennte Schichten auf der festen Komponente vorhanden sind. Vielmehr ist einsichtig, daß abhängig von der Mikrostruktur der festen Komponente eine gewisse Durchmischung der Schicht des unlöslichen Proteins und des biochemischen Reagenz stattfindet. Die erfindungsgemäße Wirkung tritt jedoch nicht ein, wenn man das Protein und das Reagenz im flüssigen Zustand mischt und dann in einem Schritt auf die Testschicht bzw. die entsprechende Komponente der Testschicht beschichtet.

Weitere Ausführungsformen der Erfindung sind der folgenden Beschreibung anhand der Figuren und mehrerer Beispiele zu entnehmen. Es zeigen:

Fig. 1    einen erfindungsgemäßen Testträger in Seitenansicht,

Fig. 2    einen Querschnitt durch eine Testschicht mit einer Partikel-Verbundstruktur.

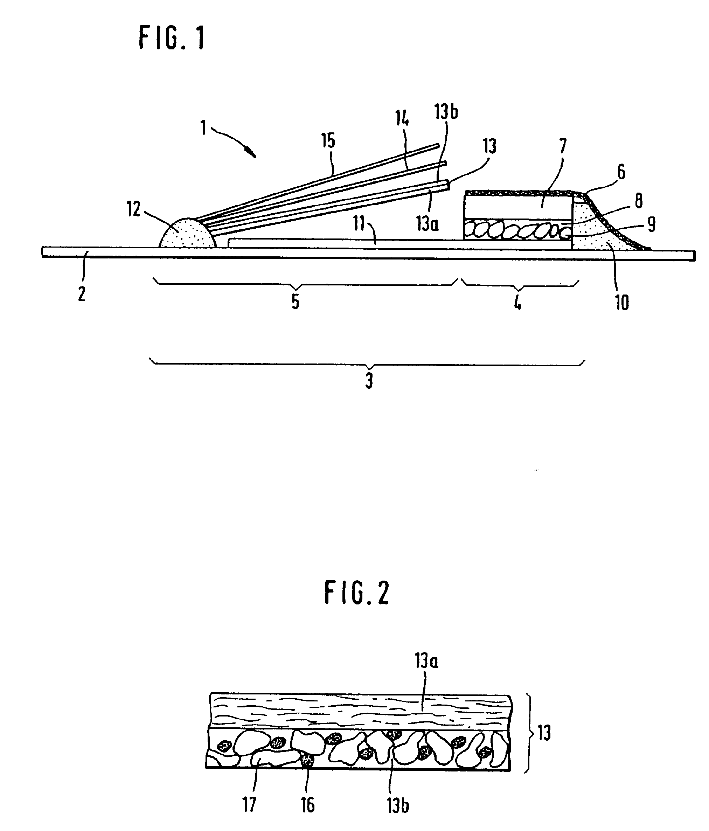

Der in Fig. 1 dargestellte Testträger 1 hat die prinzipielle Form eines Teststreifens.Es handelt sich jedoch um ein mit den früheren Teststreifen kaum mehr vergleichbares hochwertiges Analysesystem, insbesondere für die Durchführung immunologischer Bestimmungen.

Auf einer Basisschicht 2 befindet sich der insgesamt mit 3 bezeichnete Testbereich, der sich nur über einen Teil der Länge der Basisschicht 2 erstreckt. Der Testbereich 3 läßt sich in eine Probenaufgabezone 4 und in eine Auswertezone 5 unterteilen.

In der Probenaufgabezone 4 erkennt man von oben nach unten ein Abdecknetz 6, eine Erythrozytenabtrennschicht 7 und eine Reagenzschicht 8, die mit einem Schmelzkleberstreifen 10 an der Basisschicht 2 befestigt sind.

Eine Flüssigkeitstransportschicht 11 aus einem saugfähigen Material, die ebenfalls mit dem Schmelzkleberstreifen 10 fixiert ist, erstreckt sich aus der Probenaufgabezone 4 in die Auswertezone 5. Über dem nicht von den Schichten 7 bis 9 bedeckten Bereich der Flüssigkeitstransportschicht 11 befinden sich drei Schichten, die mit einem Schmelzkleberstreifen 12 an der Basisschicht 2 derartig befestigt sind, daß sie ohne äußeren Druck schräg von dieser abstehen und sie nicht berühren. Es handelt sich um eine Testschicht 13 mit einem trägerfixierten immunologischen Reagenz, um eine Reagenzschicht 14 und um eine Abdeckfolie 15.

Der dargestellte bevorzugte Testträger ist insbesondere zur Durchführung immunologischer Bestimmungen geeignet, die dem sogenannten IEMA-Prinzip nachgebildet sind. Soll beispielsweise ein in der Probe enthaltenes Antigen (Ag) bestimmt werden, so läuft die Analyse folgendermaßen ab:

Ein Tropfen Blut (etwa 30 µl) wird oberhalb der Erythrozytenabtrennschicht auf das Abdecknetz 6 aufgegeben und durchdringt die Erythrozytenabtrennschicht 7, die beispielsweise gemäß dem US-Patent 4 477 575 ausgebildet sein kann. Das so erhaltene Serum dringt in die Schicht 8 ein.

Die Schicht 8 besteht aus einem mit Reagenz imprägnierten Gewebe 9. Sie enthält einen Antikörper (Ak) für das Ag, der enzymatisch markiert und im Überschuß gegenüber der maximalen Ag-Konzentration in der Probe vorhanden ist. Dieses Antikörper-Enzym Konjugat (AkE) wird von dem eindringenden Serum gelöst.Dabei bilden sich Komplexe zwischen dem AkE und dem Ag, die mit Ag-AkE bezeichnet werden. Da das AkE im Überschuß vorhanden ist, bleibt bei Einstellung des Gleichgewichts freies Konjugat AkE übrig.

Aufgabe der Schicht 13 ist es, dieses den weiteren Nachweis störende AkE durch eine immunologische Bindung von den Ag-AkE Komplexen zu trennen. Sie wird deshalb auch als immunologische Trennschicht bezeichnet. Sie enthält den Analyt oder ein analyt-analoges Antigen in trägerfixierter Form.

Nach Ablauf einer vorbestimmten Inkubationszeit, in der sich das Gleichgewicht in der vorausgehenden Reaktion eingestellt hat, wird von oben ein Druck auf die Schichten 13 - 15 ausgeübt. Dies kann manuell oder -wie beispielsweise in der EP-A- 129 220 beschrieben- mit Hilfe eines Gerätebauteils mechanisch geschehen. Durch das Andrücken kommt die immunologische Abtrennschicht 13 mit der Flüssigkeitstransportschicht 11 in Kontakt und die darin enthaltenen Bestandteile dringen in die Schicht 13 vor. Dabei koppeln die nicht komplexierten AkE an das fixierte Ag an. Die Ag-AkE Komplexe können dagegen ungehindert weiter vordringen, soweit sie nicht durch unerwünschte unspezifische Bindungen festgehalten werden.

Die Reagenzschicht 14 enthält ein farbbildendes Substrat für das Markierungsenzym. Wenn die Flüssigkeit das Substrat erreicht hat, katalysiert das Enzym der freien Ag-AkE Komplexe die Farbreaktion des Substrats. Die Änderungsgeschwindigkeit des Farbumschlages ist deswegen ein Maß für die bei der Reagenzschicht 14 angelangten freien Komplexe Ag-AkE. Diese wiederum sind ein Maß für das in der Probe enthaltene Ag. Bezüglich des Ablaufs eines IEMA Tests auf einem Testträger wird ergänzend auf die deutsche Patentanmeldung 36 38 654 Bezug genommen.

Bei dem dargestellten Testträger ist die erfindungsgemäße Proteinbeschichtung in der Reagenzschicht 8 realisiert.

Die Schicht 8 ist eine Reagenzträgerschicht welche zugleich einen Flüssigkeitstransport von der Schicht 7 in die Schicht 11 bewirkt. Das Gewebe 9 bildet die feste Komponente, die zunächst mit dem unlöslichen Protein und dann mit dem Reagenz, in diesem Falle dem löslichen Antikörper-Enzym-Konjugat, beschichtet ist. Dadurch wird eine weitgehend vollständige Eluation des Reagenz auch nach langer Lagerzeit erreicht.

Bei der immunologischen Abtrennschicht 13 steht die Transportfunktion im Vordergrund, obwohl die Schicht zugleich als Reagenzträger (für das trägerfixierte Ag) dient. Diese Schicht ist für die Funktion des Testträgers von besonderer Bedeutung, weil die Farbänderung in der Substratschicht 14 nur dann ein reproduzierbares Maß für die Ag-Konzentration ist, wenn die Schicht 13 für eine vollständige Abtrennung der nicht komplexierten AkE sorgt und zugleich die Ag-AkE-Komplexe vollständig oder zumindest in einem gleichbleibenden Maße durchläßt.

Der prinzipielle Aufbau einer zweckmäßigen Abtrennschicht ist in Fig. 2 schematisch dargestellt. Sie besteht aus einem tragenden Gewebe oder Vlies 13 a und einer darauf angebrachten Beschichtung in Form einer Partikel-Verbundstruktur 13b.

Die Partikel-Verbundstruktur 13b enthält Reagenzträgerteilchen 16, die im beschriebenen Beispielsfall das trägerfixierte Antigen tragen. Außerdem enthält sie Öffnerteilchen 17. Die Teilchen 16 und 17 bilden zwei feste Komponenten der Schicht 13b. Sie werden durch ein geeignetes Klebemittel, das in der Figur der Übersichtlichkeit halber nicht dargestellt ist, zu einer dreidimensionalen offenporigen Schichtstruktur verbunden.

Beispiel 1:

Demonstration der verbesserten Verfügbarkeit eines biochemischen Reagenz durch Casein-Vorbehandlung im Vergleich zum Stand der Technik.

Casein-Vorbehandlung:

300 cm$^2$ eines multifilen Polyestergewebes (2F777, Schweizer Seidengazefabrik Thal, Schweiz) werden 30 Minuten lang mit folgender Lösung behandelt:
0,1 % Casein, 0,1 % Triton X 100® in Wasser, mit 1 N NaOH auf pH 11,5 eingestellt.
Anschließend wird das Gewebe mit 0,15 M Na-Phosphat-Puffer pH 7,0 neutral gewaschen und 30 Min. bei 50°C getrocknet.

Auf dieses Casein-vorbehandelte sowie auf unbehandeltes Gewebe wird ein anti-Theophyllin-IgG-β-Galactosidase-Konjugat mit den folgenden Varianten getränkt und jeweils 1 Stunde bei 40°C getrocknet.

a) Das Konjugat wird in PBS (0,15 M NaCl, 0,01 M Na-Phosphat pH 7,4) mit 0,1 % Triton X 100® auf 50 U/ml Enzymaktivität eingestellt und auf das unbehandelte Gewebe getränkt.

b) Das Konjugat wird in PBS mit 0,1 % Triton X 100®, 5mg/ml Rinderserumalbumin und 2 % Trehalose auf 50 U/ml Enzymaktivität eingestellt und auf das unbehandelte Gewebe getränkt.

c) Das Konjugat wird in PBS mit 0,1 % Triton X 100® auf 50 U/ml Enzymaktivität eingestellt und auf Casein-vorbehandeltes Gewebe getränkt.

d) Das Konjugat wird in PBS mit 0,1 % Triton X 100, 5mg/ml Rinderserumalbumin und 2 % Trehalose auf 50 U/ml Enzymaktivität eingestellt und auf das Casein-vorbehandelte Gewebe getränkt.

Die Gewebestücke a) bis d) werden in Aluminiumröhren mit Trockenmittel bis zu 6 Wochen bei verschiedenen Temperaturen (4°, 25°, 45°) aufbewahrt.

Zu verschiedenen Zeitpunkten wird die noch vorhandene Enzymaktivität untersucht.

Vergleichende Bestimmung der Enzymaktivität

6 X 6 mm große Gewebestücke werden mit 100 µl PBS 20 sec unter Schütteln eluiert.

In Mikrotiterplatten werden 50 µl des Eluates zu 200 µl Substratlösung ( 1 mM Chlorphenolrotgalactosid in 25 mM NaCl, 5 mM MgCl$_2$, 20 mM HEPES, NaOH ad pH 7,2) gegeben.

Zu verschiedenen Zeiten werden die Extinktionen gemessen und in mE/min umgerechnet.
Die Ergebnisse der Temperaturbelastungsuntersuchungen sind in der folgenden Tabelle aufgeführt.

Einheit: mE / 10 min

| Variante | Belastungszeit (Wochen) | 4°C | 25°C | 45°C |
|---|---|---|---|---|
| a) | 0 | 698 | 698 | 698 |
| ohne Zusatz | 2 | 682 | 672 | 569 |
| | 4 | 670 | 621 | 431 |
| | 6 | 559 | 428 | 173 |
| b) | 0 | 705 | 705 | 705 |
| Trehalose | 2 | 697 | 670 | 653 |
| Albumin | 4 | 675 | 635 | 551 |
| | 6 | 598 | 544 | 361 |
| c) | 0 | 710 | 710 | 710 |
| Casein | 2 | 693 | 730 | 680 |
| | 4 | 711 | 729 | 730 |
| | 6 | 697 | 695 | 705 |
| d) | 0 | 720 | 720 | 720 |
| Casein | 2 | 722 | 740 | 708 |
| Trehalose | 4 | 698 | 732 | 719 |
| Albumin | 6 | 703 | 735 | 726 |

Man erkennt, daß die Verfügbarkeit des Konjugats nach der Temperaturbelastung bei den Casein-beschichteten Varianten nahezu vollständig ist, während sie bei den nicht mit Casein beschichteten Varianten durch die Belastung sehr stark vermindert wird.

Erfahrungsgemäß ist bei einer Haltbarkeit von 6 Wochen bei 45°C, wie in den erfindungsgemäßen Varianten c) und d) gegeben, eine Haltbarkeit von mindestens 2 Jahren bei Raumtemperatur zu erwarten.

Die angegebenen Meßwerte sind Mittelwerte aus jeweils 6 Einzelwerten. Es handelt sich um Labormuster mit verhältnismäßig großen Streuungen (vK 5-10 %).

Beispiel 2:

Verfügbarkeitssteigernde Wirkung der Casein-Vorbehandlung auf pure β-Galactosidase sowie verschiedene β-Galactosidase-Konjugate.

600 cm$^2$ eines Polyestervlieses (DU Pont, Reemay 2033) werden, wie in Beispiel 1, mit Casein belegt und getrocknet.

Je 1/4 des Vlieses wird mit den folgenden Proben getränkt:

a) ein hochmolekulares (Molmasse größer 10 Millionen) anti-Theophyllin-IgG-β-Galactosidase-Konjugat;
b) ein niedermolekulares (Molmasse kleiner 2,5 Millionen) anti-Theophyllin-Fab-β-Galactosidase-Konjugat
c) ein Tetrajod-thyronin-β-Galactosidase-Konjugat (als Beispiel für ein Hapten-β-Galactosidase-Konjugat)
d) unmodifizierte β-Galactosidase.

Es werden jeweils Lösungen mit 50 U/ml Enzymaktivität, 0,1 % Triton X 100®, 5 mg/ml Rinderserumalbumin und 2 % Trehalose in PBS hergestellt, getränkt und 1 Stunde bei 40°C getrocknet.

Die Gewebestücke werden in Aluminiumröhren mit Trockenmittel 6 Wochen bei 45°C aufbewahrt.

Danach wird die noch vorhandene Enzymaktivität bestimmt: (Mittelwerte aus 6 Einzelwerten, vK ca. 8 %).

| (mE10/min) | Anfangsaktivität | Aktivität nach 6 Wochen 45°C | % Restaktivität |
|---|---|---|---|
| HM-IgG-Konjugat | 636 | 634 | 99 |
| NM-Fab-Konjugat | 610 | 622 | 100 |
| T$_4$-Gal-Konjugat | 574 | 554 | 96 |
| ß-Galactosidase | 600 | 619 | 100 |

Beispiel 3:

Vermeidung unspezifischer Bindungen durch Beschichtung von Celatom mit Casein
   a) Caseinbeladung
      Eine 0,5 %ige Casein Suspension (Serva, Heidelberg, Bundesrepublik Deutschland) in dest. Wasser wird mit 2 M NaOH auf pH 12 gestellt. In 3,5 ml dieser Lösung werden 0,5 g Celatom eingetragen. Nach 30 min rühren wird die Suspension mit HCl auf pH 5 zurückgestellt; das Casein fällt aus. Es wird über eine G3 - Fritte (16-40 µm Porengröße) abgesaugt und mit PBS neutral gewaschen. Es wird bei 45°C getrocknet oder lyophilisiert.
   b) Unspezifische Bindung von (T4) Gal-Konjugat an unbeladenes und Casein beladenes Celatom
      Jeweils 100 µl (T4) Gal-Konjugat (10 IU/ml) werden mit 100 µl einer 20%igen Celatom Suspension in PBS (phosphate buffered solution)-Puffer gemischt, wobei das Celatom einmal gemäß a) hergestellt und einmal unbehandelt ist. Nach 5 min Inkubation bei Raumtemperatur wird abzentrifugiert, der Überstand abgenommen und die Enzymaktivität in ΔE/min im Überstand bestimmt. Als Kontrolle wurden statt der Celatom Suspension 100 µl PBS mitgeführt.

| | Kontrolle | Celatom unbeladen | Celatom caseiniert |
|---|---|---|---|
| ΔE/min | 1,246 | 0,024 | 1,227 |
| % Aktivität im Überstand | 100 | 1,9 | 98,5 |
| % unspezifische Bindung | -- | 98,1 | 1,5 |

**Patentansprüche**

1.  Testträger für die Analyse einer Probenflüssigkeit, insbesondere einer Körperflüssigkeit mit einer porösen Testschicht (8),
    deren dreidimensionale offenporige Struktur von mindestens einer festen Komponente der Testschicht gebildet wird und
    die ein biochemisches Reagenz in eluierbarer Form und definierter Menge enthält,
    **dadurch gekennzeichnet,**
    daß die mindestens eine feste Komponente eine durch präzipitation eines in der Probenflüssigkeit unter Testbedingungen unlöslichen Proteins gebildete Proteinbeschichtung aufweist,
    wobei die Proteinbeschichtung und das eluierbare Reagenz in zwei getrennten Schichten aufgebracht sind.

2. Testträger nach Anspruch 1, **dadurch gekennzeichnet,**
daß das Protein Casein ist.

3. Testträger nach Anspruch 1, **dadurch gekennzeichnet,**
daß das Reagenz ein Enzym, insbesondere Galactosidase oder ein Galactosidase-Konjugat ist.

4. Testträger nach Anspruch 1, **dadurch gekennzeichnet,**
daß die dreidimensionale offenporige Struktur der Testschicht (8) als textile Struktur, bevorzugt aus Polyester oder Polyamid, ausgebildet ist.

5. Verfahren zur Herstellung einer Testschicht für einen Testträger nach Anspruch 1, **dadurch gekennzeichnet,** daß man
in einem ersten Schritt das Protein in einem Lösungs- mittel unter Bedingungen löst, bei denen die Löslichkeit des Proteins ausreichend hoch ist, um eine bestimmte Mindestmenge des Proteins zu lösen, die feste Komponente der Testschicht mit der Lösung kontaktiert und die Löslichkeit so weit vermindert, daß die Komponente von dem ausfallenden Protein beschichtet wird
und in einem zweiten Schritt das eluierbare biochemische Reagenz aufbringt.

6. Verfahren nach Anspruch 5, **dadurch gekennzeichnet,**
daß man die Löslichkeit durch Änderung des pH-Wertes, insbesondere durch Anhebung auf mindestens pH = 10,5, ändert.

## Claims

1. Test carrier for the analysis of a liquid sample, especially of a body fluid, which has a porous test layer (8),
whose three-dimensional, open-pored structure is formed by at least one solid component of the test layer and
which contains a biochemical reagent in an elutable form and in a definite amount,
**characterized in that**
the at least one solid component comprises a protein covering layer which is formed by precipitation of a protein which is not soluble in the liquid sample under test conditions,
wherein the protein layer and the elutable reagent are coated in two separate layers.

2. Test carrier according to claim 1, characterized in that the protein is casein.

3. Test carrier according to claim 1, characterized in that the reagent is an enzyme, especially galactosidase or a galactosidase conjugate.

4. Test carrier according to claim 1, characterized in that the three-dimensional open-pored structure of the test layer is formed by a textile structure, preferably made of polyester or polyamide.

5. Process for producing a test layer for a test carrier according to claim 1, characterized in that
in a first step the protein is dissolved in a solvent under conditions under which the solubility of the protein is sufficiently high in order to dissolve a certain minimum amount of the protein, the solid component of the test layer is contacted with the solution and the solubility is reduced to such an extent that the component is coated by the precipitating protein and
in a second step the eluable biochemical reagent is coated on.

6. Process according to claim 1, characterized in that the solubility is changed by changing the pH value, especially by increasing the pH value to at least pH = 10,5.

## Revendications

1. Diagnostique pour l'analyse d'un échantillon liquide, en particulier d'une humeur, comportant une couche de test poreuse (8)

dont la structure tridimensionnelle à pores ouverts est constituée d'au moins un composant solide de la couche de test et

qui contient un réactif biochimique éluable et en quantité définie,

**caractérisé en ce que**

ledit composant solide présente un enduit de protéine formé par la precipitation d'une protéine qui, dans les conditions de test est insoluble dans l'échantillon liquide,

ledit revêtement de protéine et le réactif éluable étant appliques en deux couches séparées.

2. Diagnostique selon la revendication 1, **caractérisé en ce que** la protéine est de la caséine.

3. Diagnostique selon la revendication 1, **caractérisé en ce que** le réactif est un enzyme, en particulier de la galactosidase ou un conjugué de la galactosidase.

4. Diagnostique selon la revendication 1, **caractérisé en ce que** la structure tridimensionnelle à pores ouverts de la couche de test (8) se présente sous forme de structure textile, de preference en polyester ou en polyamide.

5. Procédé pour la préparation d'une couche de test pour le diagnostique selon la revendication 1, **caractérisé en ce que,**

l'on dissout, dans une première étape, la protéine dans un solvant dans des conditions assurant un degré de dissolution de la protéine suffisamment élevé pour pou voir en dissoudre une quantité minimum déterminée, l'on met en contact le composant solide de la couche de test avec la solution et l'on réduit la solubilité de maniè- re à ce que le composant soit enduit de la protéine précipitée et que

l'on applique, dans une deuxième étape, le réactif biochimique éluable.

6. Procédé selon la revendication 5, **caractérisé en ce que** l'on modifie la solubilité en faisant varier la valeur pH, en particulier en la portant à au moins 10,5.

FIG. 1

FIG. 2